# EUROPEAN PATENT SPECIFICATION

(11) **EP 3 015 109 B1**
(45) Date of publication and mention of the grant of the patent: **18.09.2019**
(21) Application number: 15191268.0
(22) Date of filing: 23.10.2015
(51) Int. Cl.: A61K 35/744, A61K 31/80, A61P 11/00

(54) **BIOLOGICAL BARRIER WITH SIMETHICONE FOR THE USE IN THE TREATMENT OF NASO-PHARYNGO-TUBAL INFECTIONS**
BIOLOGISCHE BARRIERE MIT SIMETHICON ZUR VERWENDUNG BEI DER BEHANDLUNG VON MUND-RACHEN-TUBEN-INFEKTIONEN
BARRIÈRE BIOLOGIQUE AVEC SIMÉTHICONE POUR L'UTILISATION DANS LE TRAITEMENT DES INFECTIONS NASO-PHARYNGO-TUBAL

(30) Priority: 28.10.2014 IT RM20140610; 10.06.2015 IT UB20151275
(43) Date of publication of application: 04.05.2016
(73) Proprietor: D.M.G. Italia Srl, 00071 Pomezia (Rome) (IT)
(72) Inventor: Mercuri, Luigi, I-00071 Pomezia (Rome) (IT); Tiberi, Licia, I-00071 Pomezia (Rome) (IT)
(74) Representative: Hoffmann Eitle

(56) References cited:
- WO-A1-99/53932
- WO-A1-2004/072272
- WO-A1-2010/056198
- WO-A1-2011/125086
- US-A1- 2004 185 032
- US-A1- 2011 268 669
- "ProbiophilusProbiotic", Internet , 29 July 2013 (2013-07-29), XP002737300, Retrieved from the Internet: URL:https://web.archive.org/web/2013062908 0242/http://herbasante.ca/2012/en/products /immune_system.php?cat=immune_system&prod= probiophilus [retrieved on 2015-03-16]
- MARIA SANTAGATI ET AL: "Bacteriocin-producing oral streptococci and inhibition of respiratory pathogens", FEMS IMMUNOLOGY AND MEDICAL MICROBIOLOGY., vol. 65, no. 1, 3 February 2012 (2012-02-03), pages 23-31, XP055176324, NL ISSN: 0928-8244, DOI: 10.1111/j.1574-695X.2012.00928.x
- MEIER R ET AL: "Review of the therapeutic use of simethicone in gastroenterology", SCHWEIZERISCHE ZEITSCHRIFT FUR GANZHEITSMEDIZIN 200711 CH, vol. 19, no. 7-8, November 2007 (2007-11), pages 380-387, XP055176909, ISSN: 1015-0684
- P. Marchisio: "Streptococcus salivarius: un probiotico topico nella prevenzione dell'otite media acuta", 33 Congresso nazionale di ANTIBIOTICOTERAPIA in età pediatrica , 29 October 2014 (2014-10-29), XP002737280, Retrieved from the Internet: URL:https://www.eiseverywhere.com/file_upl oads/9a220abe4723ae8ba4e989f8c0b5cd09_Marc hisio.pdf [retrieved on 2015-03-13]
- Anonymous: "Rinogermina, confronta prezzi e offerte rinogermina su Trova Prezzi", , 28 April 2014 (2014-04-28), XP055247802, Retrieved from the Internet: URL:http://www.trovaprezzi.it/prezzo_prodo tti-salute_rinogermina.aspx [retrieved on 2016-02-05]
- POPOVA M ET AL: "Beneficial effects of probiotics in upper respiratory tract infections and their mechanical actions to antagonize pathogens", JOURNAL OF APPLIED MICROBIOLOGY, WILEY-BLACKWELL PUBLISHING LTD, GB , vol. 113, no. 6 1 December 2012 (2012-12-01), pages 1305-1318, XP002711684, ISSN: 1364-5072, DOI: 10.1111/J.1365-2672.2012.05394.X Retrieved from the Internet: URL:http://onlinelibrary.wiley.com/doi/10. 1111/j.1365-2672.2012.05394.x/abstract [retrieved on 2012-08-03]

## Description

### Scope of the invention

The present specification discloses prophylactic and therapeutic treatment of infections of the nasopharyngeal district (frequent colds, sinusitis, tonsillitis, diseases related to adenoid hypertrophy, respiratory allergies) and, in particular, of the middle ear (otitis, tubal-tympanitis, recurrent acute otitis media).

### Background Art

Acute Otitis Media (AOM) is the most common bacterial infection of childhood. The peak incidence is at the age of 1-2 years. The risk rate of developing a new episode within one month after the onset of the first infection is 35%. Children susceptible to otitis media are approximately 5% of patients who reported three or more episodes in a year.

The treatment of these infections requires the use of anti-inflammatories or antibiotics for topical or oral use, in considerable amount and for a prolonged time, especially in the case of recurrent episodes.

The main pathogens related to the disease are *Streptococcus pneumoniae, Haemophilus influenzae and Moraxella catharralis.* These bacteria colonize the nasopharynx and spread to the tympanic cavity.

It has been observed that about 75% of the bacteria associated with episodes of recurrent otitis media is due to infections by new bacterial strains, while the remaining 25% is due to re-infection with the same strain or to unsuccessful treatment.

Furthermore, antibiotic resistance is one of the most serious public health problems worldwide. The excessive use of antibiotics, especially in Western countries, has resulted in the selection of strains resistant to the treatments, some of which even in all classes of antibiotics currently available. The increasing difficulty in finding new effective antibiotic molecules directed research interest towards alternative therapeutic approaches, which potentially offer greater specificity and safety, lack of drug interactions and ability to leverage complementary mode of action than antibiotics, drastically reducing the risk of the development of further resistance. Therefore, prevention of naso-pharyngo-tubal diseases in general, and in particular of OMA, is now a primary goal of the pediatric medical care.

Recently, the importance of the normal microbiota in the protection against the infections of the upper respiratory tract has been demonstrated, and the lack of interfering bacteria able to inhibit the growth of common pathogens of the ear, namely α-streptococci, has been associated with a higher incidence of re-infection in patients with streptococcal pharyngeal-tonsillitis. Minor amounts of α-streptococci were found in the nasopharynx of children susceptible to otitis media than non- susceptible, and in patients with secretory otitis media compared to healthy children. Experimental studies have shown that the administration of streptococci by spray, such as *Streptococcus mitis, Streptococcus sanguinis, Streptococcus oralis,* in patients with Acute Otitis Media interferes and/or inhibits the growth of pathogenic microorganisms responsible for the disease.

A probiotic strain specifically used for oral hygiene and against halitosis, *S. salivarius* K12, has been orally administrated to a group of children suffering from OMA through a composition comprising; the study has revealed that in a small percentage of treated patients the strain *S. salivarius* K12 can colonize the upper respiratory improving the symptoms of the disease [Power D.A. et al. Preliminary investigations of the colonisation of upper respiratory tract tissues of infants using a paediatric formulation of the oral probiotic Streptococcus salivarius K12. Eur J Clin Microbiol Infect Dis. 2008, 27: 1261-1263].

Studies carried out by Santagati et al. has allowed the selection of a new strain of *S. salivarius* as potential biologic barrier against pathogens in the respiratory tract. [Santagati et al. Bacteriocin-producing oral streptococci and inhibition of respiratory pathogens. FEMS Immunol Med Microbiol. 2012.65:23-31]. The new strain was isolated from the nasopharyngeal tract of a healthy volunteer. The new bacterial strain belongs to the species *Streptococcus salivarius,* and has been deposited at the Leibniz-Institut DSMZ-Deutsche Sammlung von Mikroorganismen und Zellkulturen GmbH at deposit number DSM 23307. Strain characterization studies have shown that *S. salivarius* DSM 23307 has a strong *in vitro* inhibitory activity against S. *pneumoniae,* is stable under different growing conditions, and has inhibitory activity to particularly virulent, multi-resistant to antibiotics serotypes responsible for invasive infections, such as strains of *S. pneumoniae* 19 A. Furthermore, the newly identified strain has inhibitory activity against *S. pyogenes* M-1 type, adhesion to HEp-2 cells (epithelial cells from human laryngeal carcinoma) up to 57%, lack of virulence genes.

The international patent application WO2011/125086 proposes the use of the bacterial *strain Streptococcus salivarius* DSM23307 to treat infections of the upper respiratory tract.

Now it has been observed that the well-demonstrated therapeutic activity of this bacterial strain can be consolidated and implemented by the addition of other active principles, which can exert a further action in respect of complications or side effects, that are established with the bacterial infection. Hence, an additional mucolytic action can be particularly useful in establishing an improved therapeutic action.

The nasal mucus, as well as the mucus of the other body districts, is a viscoelestic substance containing 3% of glycoprotein mucin, 2% of lipids and electrolytes and the remaining 95% of water. The high hydration level of the mucus is due to the ability of the oligosaccharide binding the mucin to attract and contain water molecules, due also to the polymerization ability of the mucin, resulting in high molecular weight chains. The mucin also presents non-glycosylated domains, bearing cysteines at the ends, forming the hydrophobic regions of the mucin and causing a distortion of the protein filament. These regions are crucial for the mucin-mucin interaction, the polymers aggregation and subsequent gelling.

Although, from a molecular point of view the interactions of the cross-linking between the mucin are not clear, it is common ground that these are based on non-covalent electrostatic and hydrophobic interactions with non-glycosylated domains, critical in determining the gelling level, the rheology/density and the volume of the nasal mucus.

Simethicone polymers, amphoteric and water soluble, due to pure physical-chemical mechanisms, are able to intercalate between the mucus glycoproteins and to alter the weak hydrophobic/electrostatic interactions. Such alteration of the mucus structure with consequent weakening of internal forces between the glycoprotein polymers and density, reduces viscosity and volume of the mucus.

Therefore, from the therapeutic point of view particularly interesting for the treatment of the bacterial infections of the upper respiratory tract is the association of simethicone with the described bacterial strain.

The US patent application No. 2011/268669 discloses the use of simethicone to treat inflammatory conditions, or inflammation of the upper respiratory tract, alone or in combination with other active ingredients.

The document "ProbiophilusProbiotic" published in the Internet page URL:https://web.archive.org/web/20130629080242/htt://he rbasante.ca/2012/en/products/immune_system.php?cat0immu ne_system&prod=probiophilus on 29.07.2013, describes a composition comprising the bacterium of the *Streptococcus salivarius* subsp. Thermophilus (R-0083) strain and simethicone.

The US patent application 2004/185032 describes the combination of probiotic and simethicone for use in the treatment of otitis media.

Following the identification of the bacterial strain S. *salivarius* DSM 23307, its therapeutic properties and its use in the pharmaceutical compositions for the treatment of infections of the respiratory tract, it is still strongly felt the need to identify particular associations of ingredients in combination with such bacterial strain bearing to the composition which they are part of an improved technical effect for the treatment and prevention of bacterial or viral ENT diseases.

### SUMMARY OF THE INVENTION

In a first aspect of the invention it relates to the association of at least the *Streptococcus salivarius* DSM 23307 bacterial strain and simethicone, and/or PEG-14 dimethicone, as active ingredient for the use in the treatment and prevention of naso-pharyngo-tubal inflammation and infection.

In a second aspect the invention relates to the composition comprising the association of at least the *Streptococcus salivarius* DSM 23307 bacterial strain and simethicone, and/or PEG-14 dimethicone, performing the local barrier function in the treatment and prevention of naso-pharyngo-tubal inflammation and infection.

In a third aspect the invention provides a product made of the composition comprising the association of the at least *S. salivarius* DSM 23307 bacterial strain and simethicone, or PEG-14 dimethicone reconstituted in a suitable solvent so as to be deliverable through an atomizer spray bottle in the naso-pharyngo-tubal district.

In a fourth aspect the invention provides a kit for easier use of the composition according to the invention by the end user.

In a further aspect the invention provides a method of prophylactic and/or therapeutic treatment of naso-pharyngo-tubal infections and/or inflammations useful, in particular, in the preventive treatment of acute otitis media in the pediatric population, namely with a history of recurrent acute otitis media.

Advantages, features and modes of employ of the present invention will become apparent from the following detailed description of some embodiments thereof, given by way of example and not of limitation.

### DETAILED DESCRIPTION OF THE INVENTION

The use of compositions comprising certain strains of *S. salivarius* in the treatment of otitis media is known from the patent application WO 2004/072272 and WO 2011/125086.

In particular, in the international patent application WO 2011/125086, the Applicant has demonstrated the effectiveness of the strain *S. salivarius* DSM 23307 as probiotic that can compete with respiratory pathogens. Compositions comprising said microorganism are a useful therapeutic tool in the treatment and prevention of infections of the upper respiratory tract. The ability to interfere with the growth of pathogenic microorganisms infections of the upper respiratory tract provides an important means of defense against pathogens. Among the various mechanisms contributing to the bacterial there is the competition for cell adhesion sites. When the indigenous bacterial flora is present on the surface to be colonized, occupying space, effectively it prevents access to the pathogen, and adhesion to the cell surface, thus blocking the colonization, growth and subsequent infection by pathogenic strains.

Now it has surprisingly been found that the association of *S. salivarius* DSM 23307 and simethicone, and/or the derivative thereof, provides the composition which comprises it with a synergistic effect such that it has an efficacy superior to that obtained by the use of compositions comprising only the bacterial strain, performing a useful action effective in the treatment and prevention of naso-pharyngo-tubal pathologies, such as acute otitis media, in particularly in patients with a history of recurrent acute otitis media.

The association according to the invention comprises at least microorganisms of the strain *S. salivarius* DSM23307 and simethicone, or the derivative thereof. The Applicant has experimentally shown that in the particular association of the invention, with a combined action, streptococcus and simethicone yields an active ingredient with increased ability to reduce the surface tension of the nasal mucus and to restore the biological protective barrier to pathogens for use in the treatment and prevention of naso-pharyngo-tubal infections.

In the invention the association of *S. salivarius* DSM 23307 and simethicone further comprise microorganisms belonging to the strain *Streptococcus oralis* 89a. *Streptococcus oralis* 89a is a normal inhabitant of the human throat, it is the predominant species in the upper respiratory tract in healthy individuals, children and adults. It adheres to the epithelial tissue of the throat, and has ability to persist in the throat, and to interfere *in vitro* and *in vivo* with different serotypes of group A streptococci (GAS).

Roos et al. have showed that in a three-month follow-up study children who had received a nasal spray comprising a composition with *S. oralis* 89a the infection relapse rate was reduced compared to the placebo administration [Roos et al. Effect of recolonisation with interfering α streptococci on recurrences of acute and secretory otitis media in children: randomized placebo controlled trial. Br. Med. J. 2001; 322:1-4] . Overall, 42% of children who had received such spray during the study period did not experience acute otitis media and at the last visit presented a normal tympanic membrane compared to 22% of children treated with placebo. In addition, 31% of treated children non-developing recurrent forms of AOM, at the last visit showed the onset of a secretory form of otitis media compared to 56% in the placebo group. In conclusion, the study of Roos et al. has showed that following the administration of α-streptococci with inhibitory activity against common pathogens of otitis media, bacterial recolonization decreased, although the level of treatment failure remained high.

The Applicant has surprisingly found that the association of the strain *S*. *salivarius* DSM 23307, *Streptococcus oralis* 89a and simethicone, and/or the derivative thereof, provides a remarkable synergistic effect to the composition comprising such a combination with an efficacy clearly superior than that obtained by the use of compositions comprising the individual components and the composition comprising the association of *S. salivarius* DSM 23307 and simethicone, performing a useful action effective and more complete in the treatment and preventive therapy of naso-pharyngo-tubal pathologies, such as acute otitis media in particularly in patients with a history of recurrent acute otitis media.

In the association according to the invention the two bacterial strains, *S. salivarius* DSM 23307 and *Streptococcus oralis* 89a, may be in the ratio of 98:2 expressed as cfu (colony forming unit).

The compositions comprising the association of streptococcus and simethicone in the two described variants, i.e. association of *S. salivarius* DSM 23307, *Streptococcus oralis 89a* and simethicone, and association of *S. salivarius* DSM 23307 and simethicone, may further comprise one or more vehicles, diluents and/or pharmaceutically acceptable excipients, and optionally, may also comprise further active ingredients.

According to the invention the bacteria are preferably provided in lyophilized form. The preparation of the composition of the invention comprising streptococci and simethicone can be implemented by mixing and dissolving the lyophilized bacterial culture in a suitable solvent.

An example of solvent suitable for the solubilisation of the lyophilized bacterial strain/s according to the invention is an isotonic buffered saline solution with pH ranging between 6.9 and 7.3, preferably the pH is 7.0, comprising the copolymer PEG/PPG (INCI: Bis-PEG13 Methoxy PEG-438/PPG-110 SMDI Copolymer), and the same flavours and simethicone; the osmolarity of the solvent is between 250 mOsm and 350 mOsm, preferably 300 mOsm. The value of pH equal to 7.0 and the osmolarity of 300 mOsm of the saline solution wherein the lyophilized powders are dissolved is achieved by the use of the buffering system consisting of sodium phosphate monobasic/sodium phosphate dibasic and sodium chloride. According to the invention it can also be used other buffering systems known to the expert of the sector. According to the invention the amount of simethicone, and or the derivative thereof, in said solvent ranges between 0.0005 and 10.00% by weight. Alternatively, in a particularly preferred embodiment of the invention the simethicone is provided as a lyophilized powder as well. The mixture of bacteria and simethicone lyophilized powder is produced through a specific procedure based on the standard method of fermentation and freeze-drying known to the experts of the sector with specific modifications and adaptations. By way of example a standard procedure for obtaining lyophilized bacteria is described in the Italian Patent No. 0001338516

Alternatively, in another particularly preferred embodiment of the composition according to the invention simethicone is provided in lyophilized form together with the microorganisms that must be reconstituted in the solvent comprising the buffered isotonic saline solution comprising the copolymer PEG/PPG (INCI name: Bis-methoxy PEG13 PEG-438/PPG-110 SMDI Copolymer) and aromas.

Providing simethicone in lyophilized form along with the microorganisms is a peculiar aspect of the invention since, being the simethicone a highly insoluble molecule in aqueous solution, this might be not miscible in the isotonic solvent buffered to pH 7.0 wherein the lyophilized bacteria is reconstituted. The co-lyophilization of streptococci and simethicone increases the bioavailability of the latter which can more contribute to the synergistic action with the bacterium to determine the final effect of the composition.

In the invention it has been used a highly soluble derivative of simethicone, namely, PEG-14 dimethicone. The final reconstituted suspension contains streptococci in an amount ranging between 3 x 10⁵ and 3 x 10¹⁴ cfu (colony forming units) per gram of composition in a volume of 10-50 ml, preferably 1-5 x 10¹¹ cfu per gram of composition in a volume of 10-15 ml.

Examples of additional excipients which can be used in the composition according to the invention are xanthan gum, carboxymethyl cellulose, silicone, petrolatum, white petrolatum, magnesium stearate, maltodextrin, mannitol, starch, glucose, glycerin, propylene glycol and the like.

Said compositions may also include carriers suitable for increasing the bioavailability, stability and tolerability of the microorganisms.

Said composition may further include carriers to further improve the adhesion of the microorganism on the mucosal surface such as the polymer PEG/PPG (Bis-Metoxy PEG 438 PEG-13/PPG-110 SMDI Copolymer), a thermo-sensitive polymer able to increase the viscosity, and hence the adhesiveness with increasing temperature.

The composition may be in any form suitable to be administered via the respiratory tract.

In the present description, for "administration via the respiratory tract" intranasal administration or inhalation is meant.

Examples of suitable forms for the administration are solutions, suspensions, emulsions, sprays, drops, ointment, gel.

Preferably, the compositions may be formulated to be administrated via the respiratory tract by a nebuliser spray with or without propellants.

Dosages and timing of intake should be determined by the physician based on the general health condition of the patient and the severity of the clinical conditions of the same.

Generally, it is recommended two sprays into each nostril or mouth daily for 7 days a month for 3/5 months.

The saline solution, wherein the lyophilized bacteria have to be dissolved appears as a clear solution, colorless, slightly viscous, with a characteristic odour, whose formulation is the following:

| Bis-methoxy PEG13 PEG438/PPG | |
|---|---|
| SMDI 110 Copolymer | 2.0% by weight |
| Sodium dihydrogen phosphate | 0.15% by weight |
| Sodium phosphate dibasic | 0.40% by weight |
| Sodium chloride | 0.60% by weight |
| PEG 14-Dimethicone | 0.25% by weight |
| Flavourings | 0.10% by weight |
| Purified water | q. s. to 100. |

This saline solution is sterilized by filtration to 0.2 µM.

Object of the present specification is also a product consisting of a heat-sealed sachet stick containing the mixture of lyophilized bacteria, i.e. the mixture of *S. salivarius* DSM 23307 and *S. oralis* 89a, or *S*. *salivarius* DSM 23307 alone, and a lyophilized antiplatelet agent, and of a vial containing 10 ml of saline solution, as described previously, wherein the lyophilized mixture is dissolved into and a nozzle spray.

In the stick heat-sealed sachet containing the mixture of lyophilized bacteria and the antiplatelet agent 2.3 g of lyophilized powder comprising at least 260 x 10⁹ cfu/g of the two bacteria together are contained, wherein *S. salivarius* DSM 23307 represents 98% in cfu and *Streptococcus oralis* 89a represents 2% in cfu. After mixing and dissolving the lyophilised powder contained in the heat-sealed sachet in the volume (10ml) of saline solution contained in the vial, each ml of the resuspended product contains at least 25 x 10⁹ cfu and each spray volume of 100 µl sprayed using the nebulizer spray provided contains at least 2.5 x 10⁹ cfu. The final solution appears as a milky solution with suspended dust and characteristic odour.

The final composition ready for use comprising the association of the bacterial strain *S. salivarius* DSM 23307, the bacterial strain *Streptococcus oralis* 89a and simethicone, or the association of the bacterial strain *S. salivarius* DSM 23307 and simethicone, according to this specification, in the above described solvent allows for the administration via the respiratory tract instant by the end user within a span of time of 7 days, wherein the bacteria in solution retain their vitality.

The invention also provides a method of treatment consisting in the at least competing in the colonization of the naso-pharyngo-tubal district by microorganisms responsible for the onset of bacterial or viral diseases. The treatment is consisting in the administration of an effective amount of the composition to a subject, or patient, in the need thereof. The treatment is addressed to determine the healing of the subject, or patient undergoing treatment, or reducing the severity of symptomatology related to infection. The treatment is carried out by administering through the respiratory tract of the patient the composition according to the invention. Dosages and timing of intake should be determined by the physician based on the general health condition of the patient and the severity of the clinical condition. In a further embodiment, the method of treatment may include a step of pre-treatment of the patient to reduce the population of the normal microflora including the particular pathogenic bacterial strain which is to be inhibited. Such pre-treatment can be carried out by antibiotic administration. An example of suitable antibiotics for this purpose is amoxicillin + clavulanic acid. Clorexidina, erythromycin, penicillin and other antibiotic agents known to the expert of the sector can be used as well.

In order to evaluate the efficacy and tolerability of the composition according to the invention a randomized double-blind, placebo-controlled study has been carried out.

The collected clinical results are now being analyzed and evaluated.

Further aspects of the invention will now be illustrated by way of example and not for limiting purposes in the examples given below.

### EXAMPLES

Example 1: Formulation of the isotonic saline solution comprising a mixture of simethicone and PEG-14 dimethicone, wherein the lyophilized microrganisms have to be reconstituted.
a) Solvent formulation:

| **Ingredient** | **Amount** |
|---|---|
| Bis-Metoxy PEG13 PEG438/PPG 110 SMDI Copolimer | 0,01-10 % w/w |
| Monobasic Sodium phosphate | q.s. a pH 7,00 e 300 mOsm |
| Dibasic Sodium phosphate | |
| Sodium chloride | |
| Simethicone/PEG 14-dimethicone | 0,0005-10,00 % w/w |
| Flavourings | 0,0001-0,50 % w/w |
| Deionized water | q.s. a 100 % w/w |

b) Lyophilized bacteria formulation (*Streptococcus salivarius* DSM 23307 only):

| **Ingredient** | **Amount** | **Weight stick** |
|---|---|---|
| *Streptococcus salivarius* DSM 23307 | 3x10⁵>3 x 10¹⁴ ufc/g | 1 - 10 g per Stick |

Or
b1) Lyophilized bacteria formulation:

| **Ingredient** | **Amount** | **Weight stick** |
|---|---|---|
| *Streptococcus salivarius* DSM 23307 and *Streptococcus oralis* 89a (98:2 cfu) | 3x10⁵>3 x 10¹⁴ ufc/g | da 1 a 10 g per Stick |

Example 2: Formulation of the isotonic saline solution without simethicone wherein the lyophilized microrganisms have to be reconstituted.
b) Solvent formulation:

| **Ingredient** | **Amount** |
|---|---|
| Bis-Metoxy PEG13 PEG438/PPG 110 SMDI Copolimer | 0,01-10 % w/w |
| Monobasic Sodium phosphate | q.s. a pH 7,00 e 300 mOsm |
| Dibasic Sodium phosphate | |
| Sodium chloride | |
| Flavourings | 0,0001-0,50 % w/w |
| Deionized water | q.s. a 100 % w/w |

b) Lyophilized bacteria formulation (*Streptococcus salivarius* DSM 23307 only):

| **Ingredient** | **Amount** | **Weight stick** |
|---|---|---|
| *Streptococcus salivarius* DSM 23307 | 3x10⁵>3 x 10¹⁴ ufc/g | 1 - 10 g per Stick |

Or
b1) Lyophilized bacteria formulation (*Streptococcus salivarius* DSM 23307 and Streptococcus oralis 89a):

| **Ingredient** | **Amount** | **Weight stick** |
|---|---|---|
| *Streptococcus salivarius* DSM 23307 and *Streptococcus oralis* 89a (98:2 cfu) | 3x10⁵>3 x 10¹⁴ ufc/g | da 1 a 10 g per Stick |

## Claims

1. Association comprising at least the bacterial strain *Streptococcus salivarius* DSM 23307, simethicone, and/or PEG-14 dimethicone, and further comprising the bacterial strain *Streptococcus oralis* 89a.

2. Association according claim 1 wherein *Streptococcus salivarius* DSM 23307 and *Streptococcus oralis* 89a are in the ratio of 98:2 expressed as cfu.

3. Association according claims 1-3, wherein the association is for use in the treatment and prophylaxis of upper respiratory tract inflammations and infections.

4. Association according claim 1 or 2, wherein the association is for use in the treatment and preventive therapy of naso-pharyngo-tubal pathologies, preferably acute otitis media.

5. Composition **characterized by** the fact to comprise the association according to claim 1 and further comprising one or more carrier agents, diluents and/or pharmaceutically acceptable excipients.

6. Composition according to claim 5, wherein *Streptococcus salivarius* DSM 23307 and *Streptococcus oralis* 89a are in the ratio of 98:2 expressed as cfu.

7. Composition according to claim 5 or 6 for medical use.

8. Composition according to claim 5 for use in the treatment of upper respiratory tract inflammations and infections.

9. Composition according claim 7 for use in the treatment and preventive therapy of naso-pharyngo-tubal pathologies, preferably acute otitis media.

10. Composition according to claims 5-6 **characterized in that** the lyophilized bacteria are dissolved in a isotonic saline solvent buffered at pH 7.0 comprising bis-Methoxy PEG13 PEG438/PPG 110 SMDI copolymer, simethicone, and/or PEG-14 dimethicone, and flavorings, having osmolarity of 300 mOsm.

11. Composition according to claim 5-6 wherein the lyophilized bacteria and simethicone, and/or PEG-14 dimethicone, are in the form of lyophilized mixture dissolved in a saline isotonic solvent buffered at pH 7.0 comprising bis-Methoxy PEG13 PEG438/PPG 110 SMDI copolymer and flavorings, having osmolarity of 300 mOsm.

12. Composition according to claims 5-6 and 10-11 wherein the final reconstituted bacterial suspension contains *streptococci* in an amount ranging between 3 x 10⁵ and 3 x 10¹⁴ cfu (colony forming units) per gram of composition in a volume of 10-15 ml.

13. Composition according to claim 6 wherein the final reconstituted bacterial suspension contains *streptococci* in an amount equal to 1-5 x 10¹¹ cfu per gram of composition in a volume of 10-15 ml.

14. Composition according to claim 10 **characterized in that** 1 to 10 g of *streptococci* (3x10⁵-3x 10¹⁴ cfu/g) are dissolved in 10-15 ml of saline isotonic solution having the formulation:
| | |
|---|---|
| bis-Methoxy PEG13 PEG438/PPG 110 SMDI copolymer | 0,01-10% w/w |
| Monobasic sodium phosphate | q.s. to pH 7,00 and 300 mOsm |
| Dibasic sodium phosphate | |
| Sodium chloride | |
| Simethicone/PEG-14 dimethicone | 0,0005-10,00% w/w |
| Flavouring | 0,0001- 0,50 % w/w |
| Deionized water | q.s. a 100 % w/w |

15. Composition according to claim 11 **characterized in that** 1 to 10 g of *streptococci* (3x10⁵-3x 10¹⁴ cfu/g) are dissolved in 10-15 ml of saline isotonic solution having the formulation:
| | |
|---|---|
| bis-Methoxy PEG13 PEG438/PPG 110 SMDI copolymer | 0,01-10% w/w |
| Monobasic sodium phosphate | q.s. to pH 7,00 and 300 mOsm |
| Dibasic sodium phosphate | |
| Sodium chloride | |
| Flavoring | 0,0001- 0,50 % w/w |
| Deionized water | q.s. a 100 % w/w |

16. Composition according to claims 5-6 and 10-15 in a form suitable for administration via the respiratory tract, for intranasal administration, for administration by inhalation.

17. Composition according to claim 16 in the form of solution, suspension, emulsion, spray, drops, ointment, gel.

18. Kit comprising:
i) 10-15 ml sterile vial containing sterile saline isotonic buffered at pH 7.0 solution comprising PEG13-methoxy PEG438/PPG 110 SMDI copolymer, simethicone, and/or PEG-14 dimethicone, and flavorings;
ii) heat-sealed sachet in stick format containing the freeze-dried association of at least the bacterial strain *Streptococcus salivarius* DSM 23307;
iii) sterilized spray atomizer with protective cap;
iv) information leaflet with instructions for assembly, use and storage of the product.

19. Kit comprising:
i) 10-15 ml sterile vial containing sterile saline isotonic buffered at pH 7.0 solution comprising copolymer PEG13-methoxy PEG438/PPG 110 SMDI, and flavorings;
ii) heat-sealed sachet in stick format containing the mixture of freeze-dried of at least the bacterial strain *Streptococcus salivarius* DSM 23307 and simethicone, and/or PEG-14 dimethicone;
iii) sterilized spray atomizer with protective cap;
iv) information leaflet with instructions for assembly, use and storage of the product.

20. Kit according to claims 18-19 wherein the heat-sealed sachet in stick format containing the mixture of freeze-dried of at least the bacterial strain *Streptococcus salivarius* DSM 23307 and simethicone, and/or PEG-14 dimethicone, further contain the bacterial strain *Streptococcus oralis* 89a in the ratio of 98:2 expressed as cfu.

## Patentansprüche

1. Kombination, umfassend mindestens den Bakterienstamm *Streptococcus salivarius DSM 23307,* Simeticon, und/oder PEG-14 Dimeticon und ferner den Bakterienstamm *Streptococcus oralis 89a.*

2. Kombination gemäß Anspruch 1, wobei Streptococcus *salivarius DSM 23307* und *Streptococcus oralis* 89a im Verhältnis 98:2, ausgedrückt als KBE, vorliegen.

3. Kombination gemäß Ansprüchen 1-3, wobei die Kombination zur Verwendung bei der Behandlung und der Prophylaxe von Entzündungen und Infektionen der oberen Atemwege vorgesehen ist.

4. Kombination gemäß Anspruch 1 oder 2, wobei die Kombination zur Verwendung bei der Behandlung und präventiven Therapie naso-pharyngo-tubaler Pathologien, vorzugsweise der akuten Otitis media, vorgesehen ist.

5. Zusammensetzung, **dadurch gekennzeichnet, dass** sie die Kombination gemäß Anspruch 1 und zusätzlich ein oder mehrere Trägermittel, Verdünnungsmittel und/oder einen oder mehrere pharmazeutisch verträgliche Hilfsstoffe umfasst.

6. Zusammensetzung gemäß Anspruch 5, wobei *Streptococcus salivarius DSM 23307* und *Streptococcus oralis* 89a im Verhältnis 98:2, ausgedrückt als KBE, vorliegen.

7. Zusammensetzung gemäß Anspruch 5 oder 6 zur medizinischen Verwendung.

8. Zusammensetzung gemäß Anspruch 5 zur Verwendung bei der Behandlung von Entzündungen und Infektionen der oberen Atemwege.

9. Zusammensetzung gemäß Anspruch 7 zur Verwendung bei der Behandlung und präventiven Therapie naso-pharyngo-tubaler Pathologien, vorzugsweise der akuten Otitis media.

10. Zusammensetzung gemäß Ansprüchen 5-6, **dadurch gekennzeichnet, dass** die lyophilisierten Bakterien in einer isotonen Kochsalzlösung gelöst sind, die bei pH 7.0 gepuffert ist und bis-Methoxy PEG13 PEG438/PPG 110 SMDI Copolymer, Simeticon und/oder PEG-14 Dimeticon und Geschmacksstoffe umfasst und eine Osmolarität von 300 mOsm aufweist.

11. Zusammensetzung gemäß Ansprüchen 5-6, wobei die lyophilisierten Bakterien und Simeticon und/oder PEG-14 Dimeticon in Form einer lyophilisierten Mischung vorliegen, die in einer isotonischen Kochsalzlösung gelöst ist, welche bei pH 7.0 gepuffert ist, bis-Methoxy PEG13 PEG438/PPG 110 SMDI Copolymer und Geschmacksstoffe umfasst und eine Osmolarität von 300 mOsm aufweist.

12. Zusammensetzung gemäß Ansprüchen 5-6 und 10-11, wobei die endgültig rekonstituierte Bakteriensuspension *streptococci* in einer Menge enthält, die im Bereich zwischen 3 x 10⁵ und 3 x 10¹⁴ KBE (koloniebildende Einheiten) pro Gramm Zusammensetzung in einem Volumen von 10-15 ml liegt.

13. Zusammensetzung gemäß Anspruch 6, wobei die endgültig rekonstituierte Bakteriensuspension *streptococci* in einer Menge enthält, die 1-5 x 10¹¹ KBE pro Gramm Zusammensetzung in einem Volumen von 10-15 ml entspricht.

14. Zusammensetzung gemäß Anspruch 10, **dadurch gekennzeichnet, dass** 1 bis 10 g *streptococci* (3x10⁵-3x10¹⁴ KBE/g) in 10-15 ml isotoner Kochsalzlösung gelöst sind, die folgende Formulierung aufweist:
| | |
|---|---|
| bis-Methoxy PEG13 PEG438/PPG 110 SMDI Copolymer | 0,01-10% w/w |
| Monobasisches Natriumphosphat | q.s. bis pH 7,00 und 300 mOsm |
| Dibasisches Natriumphosphat | |
| Natriumchlorid | |
| Simeticon/PEG-14 Dimeticon | 0,0005-10,00% w/w |
| Geschmacksstoffe | 0,0001- 0,50% w/w |
| Deionisiertes Wasser | q.s. ad 100% w/w |

15. Zusammensetzung gemäß Anspruch 11, **dadurch gekennzeichnet, dass** 1 bis 10 g *streptococci* (3x10⁵-3x 10¹⁴ KBE/g) in 10-15 ml isotonischer Kochsalzlösung gelöst ist, die folgende Formulierung aufweist:
| | |
|---|---|
| bis-Methoxy PEG13 PEG438/PPG 110 SMDI Copolymer | 0,01-10% w/w |
| Monobasisches | |
| Natriumphosphat | q.s. bis pH 7,00 und 300 mOsm |
| Dibasisches Natriumphosphat | |
| Natriumchlorid | |
| Geschmacksstoffe | 0,0001- 0,50% w/w |
| Deionisiertes Wasser | q.s. ad 100% w/w |

16. Zusammensetzung gemäß Ansprüchen 5-6 und 10-15 in einer Form, die für die Verabreichung über die Atemwege, für die intranasale Verabreichung, für die inhalative Verabreichung geeignet ist.

17. Zusammensetzung gemäß Anspruch 16 in Form einer Lösung, einer Suspension, einer Emulsion, eines Sprays, von Tropfen, einer Salbe, eines Gels.

18. Kit, umfassend:
i) sterile 10-15 ml Ampulle mit einer sterilen isotonischen Kochsalzlösung, die bei pH 7.0 gepuffert ist und PEG13-methoxy PEG438/PPG 110 SMDI Copolymer, Simeticon und/oder PEG-14 Dimeticon und Geschmacksstoffe umfasst;
ii) heißversiegeltes Sachet im Stick-Format, welches zumindest den lyophilisierten Bakterienstamm *Streptococcus salivarius* DSM 23307 enthält;
iii) sterilisierter Sprühzerstäuber mit Schutzkappe;
iv) Informationsblatt mit Anweisungen für das Zusammensetzen, für die Verwendung und die Lagerung des Produktes.

19. Kit, umfassend:
i) sterile 10-15 ml Ampulle mit einer sterilen isotonischen Kochsalzlösung, die bei pH 7.0 gepuffert ist und PEG13-methoxy PEG438/PPG 110 SMDI und Geschmacksstoffe umfasst;
ii) heißversiegeltes Sachet im Stick-Format, welches die lyophilisierte Mischung aus zumindest dem Bakterienstamm *Streptococcus salivarius* DSM 23307 und Simeticon und/oder PEG-14 Dimeticon enthält;
iii) sterilisierter Sprühzerstäuber mit Schutzkappe;
iv) Informationsblatt mit Anweisungen für das Zusammensetzen, für die Verwendung und für die Lagerung des Produktes.

20. Kit gemäß Ansprüchen 18-19, wobei das heißversiegelte Sachet im Stick-Format, das die lyophilisierte Mischung aus zumindest dem Bakterienstamm *Streptococcus salivarius* DSM 23307 und Simeticon und/oder PEG-14 Dimeticon enthält, zusätzlich den Bakterienstamm *Streptococcus oralis* 89a im Verhältnis 98:2, ausgedrückt als KBE, enthält.

## Revendications

1. Association comprenant au moins la souche bactérienne *Streptococcus salivarius* DSM 23307, de la siméthicone, et/ou de la diméthicone PEG-14, et comprenant en outre la souche bactérienne *Streptococcus oralis* 89a.

2. Association selon la revendication 1, dans laquelle *Streptococcus salivarius* DSM 23307 et *Streptococcus oralis* 89a sont en un rapport de 98/2, exprimé par les ufc.

3. Association selon les revendications 1-2, laquelle association est pour une utilisation dans le traitement et la prophylaxie d'inflammations et d'infections des voies respiratoires supérieures.

4. Association selon la revendication 1 ou 2, laquelle association est pour une utilisation dans le traitement et la prophylaxie de pathologies naso-pharyngo-tubales, de préférence d'une otite moyenne aiguë.

5. Composition **caractérisée en ce qu'**elle comprend l'association selon la revendication 1, et comprenant en outre un ou plusieurs véhicules, diluants et/ou excipients pharmaceutiquement acceptables.

6. Composition selon la revendication 5, dans laquelle *Streptococcus salivarius* DSM 23307 et *Streptococcus oralis* 89a sont en un rapport de 98/2, exprimé par les ufc.

7. Composition selon la revendication 5 ou 6 pour un usage médical.

8. Composition selon la revendication 5 pour une utilisation dans le traitement d'inflammations et d'infections des voies respiratoires supérieures.

9. Composition selon la revendication 7 pour une utilisation dans le traitement et la prophylaxie de pathologies naso-pharyngo-tubales, de préférence d'une otite moyenne aiguë.

10. Composition selon les revendications 5-6, **caractérisée en ce que** les bactéries lyophilisées sont dissoutes dans un solvant de type solution salée isotonique tamponné à un pH de 7,0 comprenant un copolymère de bis-méthoxy PEG 13 PEG438 / PPG 110 SMDI, de la siméthicone, et/ou de la diméthicone PEG-14, et des aromatisants, ayant une osmolarité de 300 mOsm.

11. Composition selon les revendications 5-6, dans laquelle les bactéries lyophilisées et la siméthicone, et/ou la diméthicone PEG-14, sont sous la forme d'un mélange lyophilisé dissous dans un solvant de type solution salée isotonique tamponné à un pH de 7,0 comprenant un copolymère de bis-méthoxy PEG 13 PEG438 / PPG 110 SMDI et des aromatisants, ayant une osmolarité de 300 mOsm.

12. Composition selon les revendications 5-6 et 10-11, dans laquelle la suspension bactérienne reconstituée contient des *Streptococci* en une quantité située dans la plage comprise entre 3 x 10⁵ et 3 x 10¹⁴ ufc (unités formant des colonies) par gramme de composition dans un volume de 10 à 15 ml.

13. Composition selon la revendication 6, dans laquelle la suspension bactérienne reconstituée finale contient des *Streptococci* en une quantité égale à 1 à 5 x 10¹¹ ufc gramme de composition dans un volume de 10 à 15 ml.

14. Composition selon la revendication 10, **caractérisée en ce que** 1 à 10 g de *Streptococci* (3 x 10⁵ à 3 x 10¹⁴ ufc/g) sont dissous dans 10 à 15 ml de solution salée isotonique ayant la formulation suivante :
| | |
|---|---|
| Copolymère de bis-méthoxy PEG13 PEG438 / PPG 110 SMDI | 0,01 à 10 % en poids/poids |
| Phosphate de sodium monobasique | q.s pour pH 7,00 et 300 mOsm |
| Phosphate de sodium dibasique | |
| Chlorure de sodium | |
| Siméthicone/diméthicone PEG-14 | 0,0005 à 10,00 % en poids/poids |
| Aromatisant | 0,0001 à 0,50 % en poids/poids |
| Eau désionisée | q.s. pour 100 % en poids/poids |

15. Composition selon la revendication 11, **caractérisée en ce que** 1 à 10 g de *Streptococci* (3 x 10⁵ à 3 x 10¹⁴ ufc/g) sont dissous dans 10 à 15 ml de solution salée isotonique ayant la formulation suivante :
| | |
|---|---|
| Copolymère de bis-méthoxy PEG13 PEG438 / PPG 110 SMDI | 0,01 à 10 % en poids/poids |
| Phosphate de sodium monobasique | q.s pour pH 7,00 et 300 mOsm |
| Phosphate de sodium dibasique | |
| Chlorure de sodium | |
| Aromatisant | 0,0001 à 0,50 % en poids/poids |
| Eau désionisée | q.s. pour 100 % en poids/poids |

16. Composition selon les revendications 5-6 et 10-15 sous une forme convenant pour une administration par les voies respiratoires, pour une administration par voie intranasale, pour une administration par inhalation.

17. Composition selon la revendication 16 sous la forme d'une solution, d'une suspension, d'une émulsion, d'un spray, de gouttes, d'une pommade, d'un gel.

18. Kit comprenant :
i) un flacon stérile de 10 à 15 ml contenant de la solution salée isotonique stérile tamponnée à pH 7,0 comprenant un copolymère de PEG13-méthoxy PEG438 / PPG 10 SMDI, de la siméthicone, et/ou de la diméthicone PEG-14, et des aromatisants ;
ii) un sachet thermoscellé au format bûchette contenant l'association lyophilisée d'au moins la souche bactérienne *Streptococcus salivarius* DSM 23307 ;
iii) un atomiseur à spray stérilisé avec un capuchon protecteur ;
iv) une notice informative avec des instructions pour l'assemblage, l'utilisation et le stockage du produit.

19. Kit comprenant :
i) un flacon stérile de 10 à 15 ml contenant de la solution salée isotonique stérile tamponnée à pH 7,0 comprenant un copolymère de PEG13-méthoxy PEG438 / PPG 110 SMDI, et des aromatisants ;
ii) un sachet thermoscellé au format bûchette contenant l'association lyophilisée d'au moins la souche bactérienne *Streptococcus salivarius* DSM 23307 et de la siméthicone et/ou de la diméthicone PEG-14 ;
iii) un atomiseur à spray stérilisé avec un capuchon protecteur ;
iv) une notice informative avec des instructions pour l'assemblage, l'utilisation et le stockage du produit.

20. Kit selon les revendications 18-19, dans lequel le sachet thermoscellé au format bûche contenant le mélange d'au moins la souche bactérienne *Streptococcus salivarius* DSM 23307 lyophilisée et de siméthicone et/ou de diméthicone PEG-14 contient en outre la souche bactérienne *Streptococcus oralis* 89a en un rapport de 98/2, exprimé par les ufc.
